# FASCICULE DE BREVET EUROPEEN

(11) **EP 2 588 078 B1**
(45) Date de publication et mention de la délivrance du brevet: **14.05.2014**
(21) Numéro de dépôt: 11727707.9
(22) Date de dépôt: 28.06.2011
(51) Int. Cl.: A61K 9/00, A61K 47/14, A61K 31/557, A61K 49/00

(54) **SYSTEME DE DELIVRANCE POLYMERIQUE D'UNE SOLUTION NON VISQUEUSE A BASE DE PROSTAGLANDINE SANS CONSERVATEUR**
POLYMERSYSTEM ZUR FREISETZUNG EINER NICHT VISKOSEN PROSTAGLANDIN LÖSUNG OHNE KONSERVIERUNGSSTOFFE
POLYMERIC SYSTEM FOR DELIVERING A PRESERVATIVE-FREE PROSTAGLANDIN-BASED NONVISCOUS SOLUTION

(30) Priorité: 29.06.2010 US 359699 P; 29.06.2010 FR 1055236
(43) Date de publication de la demande: 08.05.2013
(73) Titulaire: Laboratoires Théa, 63100 Clermont Ferrand (FR)
(72) Inventeur: MERCIER, Fabrice, F-63000 Clermont-Ferrand (FR)
(74) Mandataire: Denjean, Eric
(86) Numéro de dépôt international: PCT/EP2011/060848
(87) Numéro de publication internationale: WO 2012/001009

(56) Documents cités:
- EP-A1- 2 127 638
- FR-A1- 2 918 891
- US-A1- 2004 082 660
- EDSMAN, K. ET AL: "Rheological evaluation and ocular contact time of some carbomer gels for ophthalmic use", INTERNATIONAL JOURNAL OF PHARMACEUTICS, vol. 137, no. 2, 28 juin 1996 (1996-06-28) , pages 233-241, XP002609728,
- BRASNU EMMANUELLE ET AL: "In vitro effects of preservative-free tafluprost and preserved latanoprost, travoprost, and bimatoprost in a conjunctival epithelial cell line", CURRENT EYE RESEARCH, IRL PRESS, OXFORD, GB, vol. 33, no. 4, 1 avril 2008 (2008-04-01), pages 303-312, XP009108708, ISSN: 0271-3683, DOI: DOI:10.1080/02713680801971857
- MCCAREY BERNARD ET AL: "In vivo corneal epithelial permeability following treatment with prostaglandin analoges with or without benzalkonium chloride", JOURNAL OF OCULAR PHARMACOLOGY AND THERAPEUTICS, MARY ANN LIEBERT, INC., NEW YORK, NY, US, vol. 23, no. 5, 17 octobre 2007 (2007-10-17), pages 445-451, XP002607567, ISSN: 1080-7683, DOI: DOI:10.1089/JOP.2007.0024

## Description

### DOMAINE DE L'INVENTION

L'invention a pour objet un collyre ou solution ophtalmique comprenant en tant que principe actif, au moins une prostaglandine, ladite solution étant exempte d'agent conservateur de type anti-microbien, en particulier du type ammonium quaternaire (par exemple chlorure de benzalkonium (BAK)).

Plus précisément et dans le cadre de l'invention, il a été développé un système de délivrance polymérique, permettant à la solution de prostaglandine d'être aussi efficace qu'une solution contenant du BAK mais qui n'en présente pas les inconvénients, du point de vue toxicologique et allergénique.

### ETAT DE LA TECHNIQUE

Les prostaglandines sont des principes actifs bien connus qui sont administrés à l'homme ou à l'animal par voie topique, sous forme de collyre, pour le traitement du glaucome. La posologie usuelle de ces formules est de 1 goutte par jour dans les deux yeux, sachant que les prostaglandines peuvent être également utilisées en association avec un deuxième agent anti-glaucomateux tel que par exemple, béta-bloquant, inhibiteur de l'anhydrase carbonique ou encore agoniste alpha-adrénergique.

Les prostaglandines présentent un premier inconvénient qui est celui de ne pas être solubles dans l'eau, de sorte que leur mise en solution au sein du collyre passe nécessairement par une étape préalable de solubilisation.

Par ailleurs, une autre contrainte pour le formulateur est de proposer une solution ophtalmique qui soit chimiquement stable dans le temps à température ambiante, en pratique sur une période comprise entre 18 et 24 mois. Une autre caractéristique requise de la solution ophtalmique est d'être stable vis-à-vis du conditionnement dans lequel elle est stockée, en particulier vis-à-vis des conditionnements en matériau plastique du type polyéthylène de base densité (PEBD).

Enfin, une dernière contrainte qui peut exister est d'éviter la contamination de la solution ophtalmique par des agents antimicrobiens, notamment dans le cas de figure où elle est conditionnée en multidoses. Toutefois, un remède à cette dernière problématique réside dans le conditionnement en unidoses ou dans un dispositif prévu à cet effet (par exemple système ABAK^{®} ou COMOD^{®}).

Ainsi et à ce jour, la plupart des solutions ophtalmiques à base de prostaglandines sur le marché renferment un agent conservateur qui, outre ses propriétés anti-microbiennes, assure également la solubilisation du principe actif et en partie sa stabilisation. Il s'agit par exemple du produit commercialisé sous la marque Xalatan^{®} par la société PFIZER associant Latanoprost et BAK à hauteur de 0,02 % en poids. Il est à noter que malgré la présence de BAK, ce collyre n'est pas stable à température ambiante et doit être stocké au froid, à une température de l'ordre de 5° C. La société ALLERGAN commercialise par ailleurs un collyre sous la marque Lumigan^{®} associant Bimatoprost et BAK à hauteur de 0,005 % en poids.

Toutefois, de nombreuses publications déconseillent l'usage de conservateur de type anti-microbien, en particulier le BAK, en ophtalmologie dans le cadre de traitements au long cours, comme c'est le cas notamment du glaucome, pour des problèmes de tolérance (voir en ce sens The New Class of Ophthalmic Agents: Here's how to choose the right prostaglandin for the each patient " by J. JAMES THIMONS, O.D., F.A.A.O. - Optometric management, issue May 2002).

Il est donc dorénavant établi que les agents conservateurs de type anti-microbien s'avèrent toxiques lors d'un usage au long cours, de sorte que l'on tend aujourd'hui à limiter leur usage en réduisant au maximum leur concentration dans les collyres, ou mieux, à les éliminer des formules.

Cette problématique a par exemple été prise en considération dans le document WO 97/29752, qui divulgue l'utilisation en lieu et place d'une partie du BAK, d'un agent non ionique du type Cremophor^{®}. Dans la formule proposée, la concentration en BAK est limitée à 0,01 % en poids, la concentration de Cremophor^{®} EL étant de 0,05 % en poids. Sur le marché, on trouve un produit dénommé Travatan^{®} associant Travoprost, BAK et Cremophor^{®}, commercialisé par la société ALCON.

Le polysorbate 80 a également été proposé dans des solutions ophtalmiques pour diminuer partiellement la concentration en BAK, comme c'est par exemple le cas du produit commercialisé sous la marque Rescula^{®} par la société NOVARTIS associant unoprostone avec un mélange BAK et polysorbate 80 représentant 0,015 % en poids de la solution.

Le document US2004/0082660 décrit, quant à lui, une solution ophtalmique dépourvue de BAK et contenant un mélange de latanoprost et de polysorbate 80. Autre art antérieur pertinent est FR 2 918 891.

Le problème que se propose de résoudre l'invention est donc de développer une formule à base de prostaglandine, qui remplisse au moins les conditions suivantes :
- qui soit dépourvue d'agent conservateur anti-microbien ;
- qui soit stable dans le temps en solution à température ambiante (dans un délai de 18 à 24 mois au minimum),
- qui soit compatible vis-à-vis du conditionnement en matière plastique dans lequel elle est usuellement stockée, en particulier les conditionnements en PEBD ;
- qui soit sensiblement aussi efficace, en termes de réduction de la pression intraoculaire (PIO), que celle disponible à base de BAK.

Un autre objectif est de proposer une formulation suffisamment fluide pour être conditionnée par technique de conditionnement aseptique de type « Blow-Fill-Seal » (unidoses).

### DESCRIPTION DE L'INVENTION

Ainsi, le Demandeur a mis au point un nouveau système de délivrance, qui permet à la prostaglandine d'agir avec une efficacité similaire à celle rapportée en présence de BAK.

En effet, outre son rôle de conservateur, le BAK, et de manière plus générale les molécules de type ammonium quaternaire, ont un effet dit « savon » qui permet une pénétration plus importante des molécules du principe actif dans les tissus de l'oeil, la contrepartie étant un effet toxique (irritation, sécheresse oculaire, inflammation, ...). Le système de délivrance mis en évidence dans le cadre de l'invention permet donc une pénétration et une activité équivalentes, via un mécanisme d'action distinct. En tout état de cause, elle ne présente pas les inconvénients des conservateurs classiques de type BAK.

Plus précisément, la présente invention concerne une solution ophtalmique qui comprend :
- au moins une prostaglandine ;
- un agent solubilisant ;
- un agent gélifiant de type carbomère ;
- un agent inhibant la polymérisation du carbomère ;
- un agent co-gélifiant / co-solubilisant.

Comme déjà dit, une telle solution est avantageusement dépourvue d'un agent conservateur de type anti-microbien, avantageusement de type ammonium quaternaire, encore plus avantageusement de chlorure de benzalkonium (BAK).

Dans la suite de la description, par « agent conservateur de type anti-microbien ou anti-microbien », on désigne un agent conservateur présentant des propriétés anti-microbiennes, c'est-à-dire un composé capable de garantir la protection de la solution ophtalmique vis-à-vis d'une éventuelle contamination microbienne. Un tel agent au sens de l'invention est à distinguer des agents conservateurs agissant sur la conservation chimique de la solution tels que par exemple des agents antioxydants comme l'EDTA.

La solution revendiquée comprend comme principe actif au moins une prostaglandine. En pratique, les prostaglandines sont dans la solution au nombre d'au moins une et sont choisies dans le groupe comprenant 17-phenyl-13,14 dihydro trinor prostaglandin F_{2α} isopropyl ester (latanoprost), 20-ethyl prostaglandin F_{2α}, (+)- fluprostenol isopropyl ester (travoprost), 17-phenyl trinor prostaglandin F_{2α} amide, 17-phenyl-13,14 dihydro trinor prostaglandin F_{2α} ethyl amide (bimatoprost), tafluprost prostaglandin F_{2α} ethanolamide, bimatoprost (freeacid)-d₄, bimatoprost-d₄ , latanoprost ethyl amide, 13,14 dihydro-15-keto-20-ethyl prostaglandin F_{2α} (unoprostone), 13,14 dihydro-15-keto-20-ethyl prostaglandin F_{2α} isopropyl ester (unoprostone isopropyl ester). Il s'agit avantageusement du latanoprost.

Selon une autre caractéristique, la concentration en prostaglandine dans la solution est comprise entre 0,002 et 0,15 % (w/v).

De manière classique, la ou les prostaglandines peuvent être associées à un second principe actif en particulier avec des agents anti-glaucomateux d'autres classes pouvant ainsi agir de manière synergique. Il peut s'agir par exemple des béta-bloquants choisis dans le groupe comprenant le maléate de timolol et le chlorure de carteolol, des inhibiteurs de l'anhydrase carbonique tels que ceux par exemple choisis dans le groupe comprenant le chlorure de dorzolamide ou encore des agonistes alpha-adrénergiques tels que par exemple le tartrate de brimonidine. Des exemples d'associations de prostaglandines et de béta-bloquants sont par exemple :
Xalacom^{®} - Pfizer : Latanoprost 0,005% + Timolol 0,5%,
Ganfort^{®} - Allergan : Bimatoprost 0,03% + Timolol 0,5%
Duotrav^{®} - Alcon : Travoprost 0,004% + Timolol 0,5%.

En pratique, l'agent anti-glaucomateux représente entre 0,1 et 0,5% (w/v) de la solution.

Comme déjà dit, une telle solution requiert la présence d'un agent dit solubilisant, apte à solubiliser la ou les prostaglandines. Cet agent solubilisant, de préférence non ionique, possède avantageusement des propriétés tensioactives. De manière privilégiée, il s'agit de l'hydroxystéarate de macrogolglycérol (huile de ricin hydrogénée et éthoxylée ou PEG-40 Hydrogenated Castor Oil; numéro CAS 61788-85-0). Un autre candidat potentiel est le polyoxyl-15-hydroxystéarate ou macrogol 15 hydroxystéarate (numéro CAS 70142-34-6), ainsi que le polysorbate 20 (numéro CAS 9005-64-5), le polysorbate 60 (numéro CAS 9005-67-8) ou le polysorbate 80 (numéro CAS 9005-65-6). La concentration de l'agent solubilisant dans la solution est typiquement comprise entre 0,1 et 20% (w/v), avantageusement entre 0,5 et 5% (w/v).

Une autre caractéristique importante de la solution ophtalmique est sa viscosité. En effet, celle-ci est avantageusement comprise entre 8 et 20 mPa.s (cP), encore plus avantageusement comprise entre 10 et 14 mPa.s (cP), lorsqu'elle est mesurée à l'aide d'un viscosimètre à mobile tournant BROOKFIELD RVDV III à 25°C. Il s'agit donc bien d'une solution qui se distingue d'un gel aqueux, typiquement caractérisé par une viscosité comprise en 400 et 800 mPa.s (cps) et qui constitue une forme retard dans le but d'obtenir une libération prolongée du principe actif.

Ainsi, la présente invention repose sur la mise au point d'un système de gélification adapté, basé sur la combinaison de deux agents gélifiants, permettant d'obtenir un système de délivrance polymérique non visqueux :
- un premier agent gélifiant, en l'occurrence un carbomère, normalement apte à polymériser et à former un gel mais dont la polymérisation, l'expansion ou le maintien sous forme de gel est inhibé par un agent dit cassant. Ledit agent inhibant la polymérisation du carbomère peut être une source d'ions sodium, avantageusement de l'EDTA de sodium, de l'acétate de sodium ou du chlorure de sodium. Il peut alternativement s'agir d'un polyol de bas poids moléculaire tel que le glycérol (voir FR 2 604 906). En outre, le carbomère est présent dans la solution en concentration maîtrisée, avantageusement comprise entre 0.05 et 0.2% (w/v). Bien évidemment, la concentration relative de l'agent inhibant est également importante : de 0,01 à 1% (w/v) dans le cas d'ions sodium, par exemple de 0,02 à 0,1% pour l'EDTA de sodium, ou de 0,5 à 3 % (w/v) dans le cas des polyols, par exemple le glycérol ;
- un second agent dit co-gélifiant / co-solubilisant qui, lui, a pour but d'assurer le niveau de viscosité recherché et potentialise la solubilisation du principe actif prostaglandine. Il s'agit typiquement de polymère de type polyéthylèneglycol (PEG) ou de type dérivés vinyliques comme l'alcool polyvinylique (PVA) ou la polyvinylpyrrolidone (PVP). Avantageusement, sa concentration est comprise entre 0.5 et 2.5 % (w/v).

Dans le cadre de l'invention, un carbomère est défini selon la pharmacopée Européenne, à savoir un « polymère réticulé de l'acide acrylique de très haute masse moléculaire relative, contenant une large proportion de groupes carboxyliques », et par son numéro CAS 9003-01-4. Il existe différents grades de carbomère utilisables dans le cadre de l'invention : 910 / 934 / 934P / 940 / 941 / 971 et 974P, avantageusement 974P.

Dans le cadre de l'invention, un PEG ou macrogol est défini selon la Pharmacopée Européenne, à savoir un « mélange de polymères de formule générale H-(OCH₂-CH₂)n-OH où n représente le nombre moyen de groupements oxyéthylène ». Le type de macrogol est défini par un nombre qui indique la masse molaire relative moyenne. Il s'agit avantageusement de PEG 4000 qui possède un numéro CAS 25322-68-3.

Bien entendu, la composition de l'invention peut contenir des additifs usuels à l'exclusion des agents conservateurs anti-microbiens. Il peut s'agir par exemple d'agents isotonisants de type non ioniques tels que des polyols (par exemple le sorbitol). Il peut également s'agir d'agents anti-oxydants ou de systèmes tampons (par exemple l'hydroxyde de sodium comme neutralisant).

Selon un mode de réalisation particulier, la composition selon l'invention est constituée des ingrédients listés dans le tableau ci-dessous, avantageusement selon la formule centésimale indiquée :

| **PRODUITS** | **FONCTIONS** | **FORMULE CENTESIMALE (g/100 ml)** |
|---|---|---|
| Carbomère 974 P | Gélifiant | 0.1 |
| Sorbitol | Isotonisant | 3.5 |
| PEG 4000 | Co-gélifiant / Co-solubilisant | 1.0 |
| EDTA | Inhibiteur agent gélifiant | 0.05 |
| Hydroxyde de sodium | Neutralisant | qsp pH = 7,0 |
| Eau PPI | véhicule | qsp 100 ml |
| Prostaglandine F2α | Principe actif | 0.005 |
| Macrogol glycerol hydroxystearate 40 | Solubilisant | 5.0 |

La formulation de l'invention peut être présentée dans des flacons à usage unique (unidose) ou dans un flacon multi-doses du type par exemple Abak^{®} ou Comod^{®} ou équivalent, de tels flacons permettant de délivrer des collyres sans conservateurs pendant plusieurs jours.

L'invention a donc pour objet un flacon à usage unique (unidose) ou multidose comprenant la solution ophtalmique précédemment décrite réalisé en PEBD de qualité EP ne contenant pas d'additifs.

En effet, la fluidité de la solution, associée à la stabilité du produit lorsqu'il est conditionné dans un récipient en PEBD, rend sa fabrication compatible avec la technologie BFS.

En outre, la solution selon l'invention est stable au moins 18 mois, voire au moins 24 mois, à température ambiante (25°C - 30°C).

Bien entendu, l'invention concerne également l'utilisation de la solution ophtalmique telle que précédemment décrite pour la fabrication d'un médicament destiné au traitement du glaucome chez l'homme ou l'animal, en particulier pour sa capacité à réduire la pression intraoculaire et/ou à assurer la neuroprotection des tissus rétiniens.

Ainsi il a été montré, dans le cadre de la présente demande, que ce système de délivrance était moins agressif pour les tissus de l'oeil que les systèmes de solubilisation de l'art antérieur, dans la mesure où la réduction de la pression intraoculaire était plus régulière et linéaire après administration de la solution.

En pratique, le collyre est administré à raison d'une goutte par jour dans chaque oeil.

L'invention a également pour objet une méthode de traitement thérapeutique du glaucome chez l'homme ou l'animal consistant à instiller la solution ophtalmique précédemment décrite à raison d'une goutte par jour dans chaque oeil.

Il s'avère que contrairement aux solutions proposées par l'art antérieur, l'administration d'une composition selon l'invention s'accompagne d'une diminution régulière et progressive de la pression intraoculaire (PIO), sans passage par une augmentation initiale transitoire de la PIO. Avec une composition selon l'invention, la diminution de la PIO débute plus tôt, avec à terme un effet au moins égal voire meilleur. Sans vouloir être lié à une quelconque théorie, cet effet sur la PIO pourrait être lié à la présence de la matrice de délivrance capable de contrecarrer ou du moins d'atténuer le caractère agressif de l'agent solubilisant. De surcroît, une composition selon l'invention est extrêmement bien tolérée. L'invention et les avantages qui en découlent ressortiront mieux des exemples de réalisation, suivants à l'appui de la figure annexée.
La figure 1 illustre les effets de 2 formulations de gouttes oculaires contenant 0.005% de latanoprost (composition EXEMPLE 1 repérée par des triangles pleins et Xalatan^{®} repérée par des ronds pleins) sur la pression intraoculaire (PIO) chez les rats.
La figure 2 rapporte les données cliniques concernant la tolérance d'une composition EXEMPLE 1 versus Xalatan^{®}: A/ symptômes oculaires subjectifs liés à l'instillation ; B/ symptômes oculaires subjectifs non liés à l'instillation ; C/ hyperémie conjonctivale globale.

### 1/ Compositions selon l'invention :

### 1a/ Composition EXEMPLE 1 :

| **PRODUITS** | **FORMULE CENTESIMALE (g/100 ml)** |
|---|---|
| Carbomère / gélifiant | 0,10 g |
| Sorbitol / isotonisant | 3,50 g |
| PEG / co-gélifiant / co-solubilisant | 1,00 g |
| EDTA / Source d'ion Na+ | 0,05 g |
| Hydroxyde de sodium 1N / Neutralisant | qsp pH = 7,0 |
| Latanoprost / Principe actif | 0,005 g |
| Macrogol glycerol hydroxystearate 40 / solubilisant | 5,00 g |
| Eau PPI / Véhicule | qsp 100 ml |

### 1b/ Composition EXEMPLE 2 :

| **PRODUITS** | **FORMULE CENTESIMALE (g/100 ml)** |
|---|---|
| Carbomère / gélifiant | 0,10 g |
| Sorbitol / isotonisant | 3,50 g |
| PVP / co-gélifiant / co-solubilisant | 2,00 g |
| Acétate de sodium / Source d'ion Na+ | 0,8 g |
| Hydroxyde de sodium 1N / Neutralisant | qsp pH = 7,0 |
| Latanoprost / Principe actif | 0,005 g |
| Macrogol glycerol hydroxystearate 40 / solubilisant | 5,00 g |
| Eau PPI / Véhicule | qsp 100 ml |

### 1c/ Composition EXEMPLE 3 :

| **PRODUITS** | **FORMULE CENTESIMALE (g/100 ml)** |
|---|---|
| Carbomère / gélifiant | 0,15 g |
| Sorbitol / isotonisant | 2,25 g |
| PVA / co-gélifiant / co-solubilisant | 0,50 g |
| Chlorure de sodium / Source d'ion Na+ | 0,25 g |
| Hydroxyde de sodium 1N / Neutralisant | qsp pH = 7,0 |
| Travoprost / Principe actif | 0,004 g |
| Macrogol 15 hydroxystearate/ solubilisant | 0,50 g |
| Eau PPI / Véhicule | qsp 100 ml |

### 2/ Caractérisation de la formulation EXEMPLE 1 :

La solution obtenue est opalescente, exempte de particules visibles, avec un pH compris entre 6,6 et 7,2 (formulation à pH neutre) et une osmolalité comprise entre 270 à 340 mosmol/kg (formulation isotonique).
La viscosité de la solution, mesurée à l'aide d'un Viscosimètre à mobile tournant BROOKFIELD (Mobile n° 00 / Chambre de mesure UL), est comprise entre 8,0 à 14,0 mPa.s à 25°C.
En outre, la solution s'est montrée stable au moins 24 mois à température ambiante (25 ou 30°C).

### 3/ Efficacité de la composition EXEMPLE 1 : Evaluation de l'efficacité de réduction de la pression intraoculaire, suite à l'administration chez des rats albinos normotendus au niveau oculaire

Les effets des gouttes oculaires de la formulation EXEMPLE 1 sur la pression intraoculaire ont été évalués chez un rat normotendu, en comparaison à des gouttes oculaires disponibles cliniquement, à savoir le produit Xalatan^{®}, chaque produit contenant du latanoprost à une concentration de 0.005%. De manière importante, le Xalatan^{®} contient de l'agent anti-microbien chlorure de benzalkonium (BAK), alors que la formulation EXEMPLE 1 n'en contient pas.

16 rats mâles albinos (Wistar) ont été impliqués dans cette étude. Ils ont été répartis en 2 groupes de 8 animaux chacun, dédiés à la formulation EXEMPLE 1 et au produit de référence Xalatan^{®} respectivement. Tous les animaux ont été traités au niveau des yeux droits avec 4 instillations de 10 µl chacune chaque 2 min, pour un total de 2 µg de latanoprost. Les mesures de pression intraoculaire (PIO) ont été réalisées à l'aide du dispositif « Tonolab rebound tonometer » sur les deux yeux, 1h, 2h, 4h, 6h et 8h après administration.

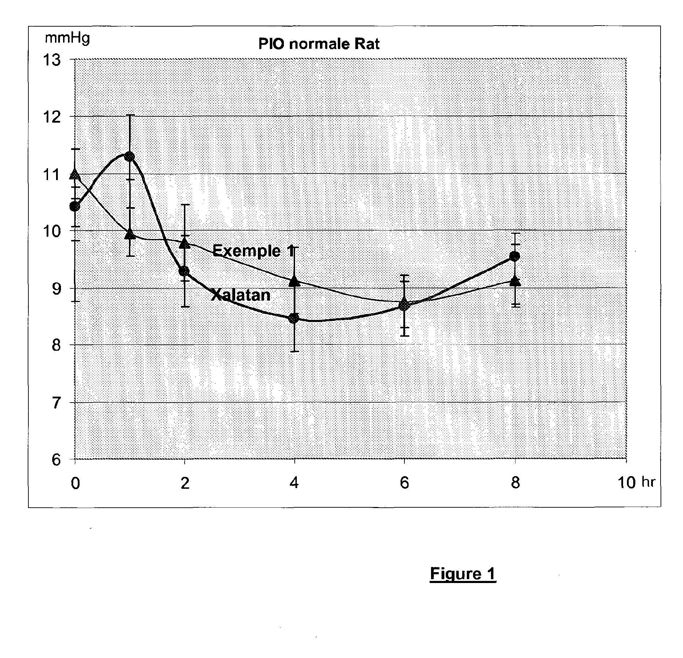

Les résultats de cette étude sont montrés à la figure 1. Chez le rat Wistar, la dose administrée de Xalatan^{®} produit un changement biphasique dans la PIO, à savoir une augmentation de pression initiale (+11,4%), avec un pic à 1h, suivi par une réponse hypotensive prolongée de 2h à 8h après traitement avec un pic de réduction de la PIO à t=4h (-18,4%). La même dose (2 µg) de latanoprost dans la formulation EXEMPLE 1 a réduit la PIO, de manière constante et progressive, pour atteindre à t=4h après l'administration topique, une réduction de 16%. Cet effet a perduré de 4h à 8h avec un pic de réduction maximal de la PIO (-20%) à t=6h.

En conclusion, il est à noter que :
- il existe une phase précoce d'hypertension qui est présente avec le Xalatan^{®} mais qui n'existe pas avec la formulation EXEMPLE 1 chez le rat normotendu ;
- la formulation EXEMPLE 1 induit une réduction de la PIO qui est relativement similaire à celle observée avec le produit de référence, cliniquement disponible, à savoir le Xalatan^{®}.
- L'absence de BAK dans la solution Exemple 1 ne compromet pas l'effet sur la réduction de la PIO.

### 4/ Tolérance oculaire de la composition EXEMPLE 1 :

La tolérance oculaire du produit EXEMPLE 1 par rapport au Xalatan^{®} a été testée à 28 jours, à raison de deux instillations quotidiennes (50 µl), chez des lapins pigmentés.
L'étude a donné les résultats suivants:
- Au niveau de la tolérance générale, tous les animaux présentaient une évolution en poids corporel normale, une bonne santé et aucun signe particulier. La consommation en eau et en nourriture étaient également normale.
- Au niveau de la tolérance oculaire, l'administration du produit EXEMPLE 1 n'a pas induit d'effets oculaires, à l'exception d'une rougeur légère et transitoire pour 1 animal sur 10. Le Xalatan^{®}, quant à lui, n'a pas induit d'effets oculaires, à l'exception d'une rougeur légère et transitoire pour 2 animaux sur 10. Quel que soit le traitement, une coloration de la cornée a été observée pour certains animaux :
   1. • EXEMPLE 1: légère à modérée pour 6 animaux sur 10 ;
   2. • Xalatan^{®}: légère pour 4 animaux sur 10 ;
   3. • Groupe contrôle: légère à modérée pour 7 animaux sur 10.
Cet effet oculaire est communément observé lors des études de tolérance impliquant des répétitions quotidiennes d'administrations topiques oculaires pendant 28 jours consécutifs.

Aucun autre effet oculaire n'a été observé pendant cette étude.
- L'administration de EXEMPLE 1 ou de Xalatan^{®} n'a pas induit d'effets macroscopiques sur les organes.
- L'analyse histologique des 2 yeux (traité et non traité) n'a révélé aucun signe pathologique, quel que soit le traitement.
- Comme pour le Xalatan^{®}, l'administration de EXEMPLE 1 n'a pas induit d'effets oculaires microscopiques.

En conclusion, suite à deux instillations quotidiennes de 50 µl de EXEMPLE 1 (latanoprost 0.005%) pendant 28 jours dans l'oeil droit de lapins pigmentés, des effets oculaires très légers ont été observés et de manière similaire dans le groupe contrôle traité avec des gouttes oculaires de Xalatan^{®} 0.005%. Les effets oculaires observés sont considérés comme non spécifiques et essentiellement dus à la répétition des instillations quotidiennes pendant 28 jours consécutifs. Par conséquent, dans ces conditions expérimentales, l'administration de EXEMPLE 1 a été très bien tolérée au niveau macroscopique et microscopique, comme pour le Xalatan^{®}.

### 5/ Essais cliniques de la composition EXEMPLE 1 :

### 5-1/EFFICACITE :

### Introduction

Une étude clinique pilote de phase II exploratoire, randomisée, investigateur masqué, multicentrique, croisée, d'une durée de 3 mois, incluant 30 patients a eu pour but de comparer la pharmacocinétique, l'efficacité et l'innocuité de la préparation ophtalmologique EXEMPLE 1 (latanoprost 0,005%) comparées à celle contenant également du latanoprost à 0,005% (Xalatan^{®}) chez des patients nouvellement diagnostiqués comme présentant un glaucome à angle ouvert ou une hypertension oculaire.
L'objectif de cette étude était de comparer les résultats obtenus suite à l'instillation d'unidose de préparation ophtalmique contenant du latanoprost (sans conservateur) et le collyre Xalatan^{®} (avec du chlorure de benzalkonium comme conservateur), après 6 semaines de traitement avec l'une des 2 options et 6 semaines avec l'autre option, chez des patients nouvellement diagnostiqués comme présentant un glaucome et une hypertension oculaire.

Cette étude a été réalisée en accord avec les standards acceptés des bonnes pratiques cliniques (Good Clinical Practice ou GCP), avec des directives spécifiques le cas échéant et conformément avec la Déclaration de Helsinki (2004) et les règlements locaux.

### Protocole

A la première visite (Visite 1), les patients ont été choisis au hasard pour recevoir un des 2 traitements suivants:
- Formulation latanoprost 0.005% avec conservateur (Multidose [MD], Xalatan^{®}) une fois par jour à 20 heures pour 6 semaines dans les deux yeux
- Formulation latanoprost 0.005% sans conservateur (Unidose [SDU], EXEMPLE 1) une fois par jour à 20 heures pour 6 semaines dans les deux yeux.
Après ces 6 semaines, les sujets ont été échangés et soumis à l'autre médication de l'étude (sans période de lavage), c'est à dire que ceux qui recevaient la première formulation avec conservateur ont reçu pendant la seconde période celle sans conservateur et vice versa. La durée de la seconde période d'étude a également été de 6 semaines.
Tous les patients ont assisté à 3 visites au centre investigateur au cours du déroulement de l'étude : la visite d'inclusion (Jour 0), la visite de suivi (Jour 42) et la visite finale (Jour 84). La PIO a été évaluée dans chaque oeil au Jour 0, Jour 42 et Jour 84, à 8h (± 30 min), 12h (± 30 min), 16h (± 30 min) et 20h (± 30 min). L'efficacité globale a été également évaluée par l'investigateur. L'innocuité et la tolérance ont également été évaluées à la fois par les patients et les investigateurs.

### Résultats

Les résultats sont présentés dans le tableau suivant :

### PIO moyenne obtenue après traitement soit avec Xalatan^{®} ou EXEMPLE 1

| PIO moyenne | Valeur de base | Après 6 semaines de traitement | | |
|---|---|---|---|---|
| | | Xalatan^{®} | EXEMPLE 1 | Xalatan^{®}-EXEMPLE 1 |
| 8 h | 22.63 | 16.20 | 16.91 | -0.71 |
| 12 h | 23.56 | 16.43 | 16.85 | -0.42 |
| 16h | 22.55 | 16.47 | 16.28 | 0.19 |
| 20h | 21.62 | 16.71 | 16.43 | 0.27 |
| Moyenne Diurne | 22.59 | 16.45 | 16.65 | -0.20 |

Les différences de PIO moyenne entre les 2 produits n'ont pas été statistiquement significatives, quel que soit le temps. La PIO diurne moyenne a été réduite de 6.14 mmHg (27.2%) après 6 semaines de thérapie avec du Xalatan^{®} et de 5.94 mmHg (26.3%) après 6 semaines de thérapie avec EXEMPLE 1. L'efficacité sur la baisse de PIO des deux traitements est donc comparable, avec obtention d'une PIO « normale » suite aux deux traitements.

En considérant les mesures au pic physiologique de la PIO à 8h: le Xalatan^{®} avait un effet réducteur de la PIO légèrement meilleur d'approximativement 0.7 mmHg. Néanmoins, cette différence n'est pas statistiquement significative (p=0.118).
De plus, cette tendance diminue tout au long de la journée pour s'inverser au cours de l'après-midi et de la soirée illustrant ainsi l'effet au cours de toute la journée de la formulation EXEMPLE 1, déjà observé au cours de l'étude d'évaluation de la réduction de la PIO chez le rats albinos normotendus.
Par conséquent, la formulation EXEMPLE 1 est efficace sur la PIO dès 8h du matin et reste tout aussi efficace tout au long de la journée et ce, malgré l'absence de conservateur.
Pour conclure, les PIO diurnes après 6 semaines des 2 traitements sont statistiquement non différentes avec une efficacité caractérisée par un maintien d'une PIO dite « normale » tout au long de la journée pour la formulation EXEMPLE 1 sans conservateur.

### 5-2/ TOLERANCE :

### Introduction

Les objectifs de cet essai clinique de phase III, en plus de son efficacité dans la réduction de la pression intraoculaire, étaient d'évaluer la tolérance et l'innocuité du collyre selon l'EXEMPLE 1 (Latanoprost 0,005 % collyre unidose sans conservateur) versus Xalatan^{®} (Latanoprost 0,005 % collyre multidose contenant des conservateurs) chez des patients atteints d'hypertonie oculaire ou de glaucome, traités par une instillation quotidienne de collyre pendant 3 mois.

### Protocole

Il s'est agi d'une étude internationale, multicentrique, randomisée, investigateur masqué, impliquant 2 groupes parallèles qui a été menée par rapport à un produit de référence chez 360 patients évaluables, traités pendant 3 mois. Une visite de sélection (J42 pour jour 42), une visite d'inclusion (J0) et 3 visites de suivi J15, J42 et J84 ont été programmées.

Un total de 463 patients ont été sélectionnés, 404 patients ont été inclus et randomisés, et 402 ont instillé le traitement objet de l'étude : 213 dans le groupe EXEMPLE 1 et 189 dans le groupe Xalatan. En tout, 392 patients ont terminé l'étude : 206 dans le groupe EXEMPLE 1 et 186 dans le groupe Xalatan.
La population retenue pour l'évaluation de la tolérance et l'innocuité des collyres était composée de 213 patients dans le groupe EXEMPLE 1 et de 189 patients dans le groupe Xalatan. L'échantillon mITTT (pour « modified Intent-to-treat » ou avec Intention de traiter modifié) était composé de 353 patients : 189 dans le groupe EXEMPLE 1 et 164 dans le groupe Xalatan. L'échantillon PP (pour « Per Protocol ») était composé de 177 patients dans le groupe EXEMPLE 1 et 153 dans le groupe Xalatan.
La population retenue pour l'évaluation de la tolérance et l'innocuité des collyres, ne différait pas significativement en termes de démographie ou d'autres caractéristiques de départ, à l'exception d'un taux un peu plus élevé de patients de sexe féminin dans le groupe EXEMPLE 1 (53,5 %) en comparaison avec le groupe Xalatan (45,5 %). L'âge variait entre 24 et 93 ans avec une moyenne ± déviation standart (DS) de 64,7 ±11,5 ans.
La population retenue pour l'évaluation de la tolérance et l'innocuité des collyres avait une épaisseur cornéenne variant entre 500 et 610 µm avec une valeur moyenne ± DS de 542 ± 22 µm dans le groupe EXEMPLE 1 et de 543 ± 22 µm dans le groupe Xalatan. La durée moyenne de traitement (en moyenne 83 jours) a été comparable pour les 2 groupes de traitement. L'observance du traitement basée sur l'instillation a été supérieure à 98 % dans chaque groupe de traitement.

### Résultats

### Evènements oculaires indésirables

Des évènements oculaires indésirables (EI) ont été rapportés chez 18 (8,5 %) patients dans le groupe EXEMPLE 1 contre 22 (11,6 %) dans le groupe Xalatan. L'événement le plus fréquent quel que soit le groupe a été l'intolérance au médicament qui a été rapportée pour 1 (0,5 %) patient dans le groupe EXEMPLE 1 contre 4 (2,1 %) patients dans le groupe Xalatan. Il y a eu un inconfort oculaire important dans le groupe EXEMPLE 1 et les autres évènements ont été d'intensité faible ou modérée.
Des EI oculaires en relation avec les médicaments étudiés ont été rapportés chez 8 (3,8 %) patients dans le groupe EXEMPLE 1 (incluant 1 patient avec 1 photophobie modérée, et 1 patient avec 1 légère hémorragie de la papille optique, pour lequel la relation n'a pas pu être prouvée) contre 10 (5,3 %) patients dans le groupe Xalatan.
Le médicament étudié a été arrêté en raison d'un EI oculaire chez 2 patients du groupe EXEMPLE 1 (une intolérance médicamenteuse modérée au jour J7 et 1 prurit oculaire modéré à J28) et chez 1 patient du groupe Xalatan (1 conjonctivite allergique modérée à J9). Tous les EI oculaires causant l'arrêt du médicament ont été reliés au médicament étudié par l'investigateur. Tous les patients ont été guéris lorsque le médicament a été arrêté. Aucun EI oculaire sévère n'a été rapporté dans cette étude.

### Evènements systémiques indésirables

Des EI systémiques ont été rapportés chez 28 (13,1 %) patients dans le groupe EXEMPLE 1 contre 32 (16,9 %) dans le groupe Xalatan. Aucun des EI systémiques n'a été relié au médicament étudié dans le groupe EXEMPLE 1 alors que 6 EI ont été rapportés chez 4 (2,1 %) patients dans le groupe Xalatan, considérés par l'investigateur comme étant reliés au traitement (2 céphalées chez le même patient, 1 vertige, 1 migraine, et 1 palpitation et 1 faiblesse musculaire). Tous étaient d'intensité mineure. Une dépression majeure dans le groupe EXEMPLE 1 et 1 migraine modérée dans le groupe Xalatan ont conduit à l'arrêt du médicament testé. 6 patients ont eu un EI sérieux (SEI) : 5 patients dans le groupe EXEMPLE 1 (1 fracture du tibia, 1 dépression majeure, 1 syncope, 1 douleur à la poitrine, et 1 fracture du péroné) et 1 patient dans le groupe Xalatan (1 colique néphrétique). Aucun n'avait de relation avec le traitement selon l'investigateur.

### Symptômes subjectifs suite à l'instillation

Globalement, les symptômes oculaires subjectifs suite à l'instillation (prurit, brûlure/picotements, sensation d'oeil collé, sensation de sécheresse oculaire, sensation d'un corps étranger) ont été significativement moins fréquents dans le groupe EXEMPLE 1 que dans le groupe Xalatan à J42 (p=0,001) et J84 (p=0,001) (voir tableau ci-dessous). Cela était dû en premier lieu au symptôme brûlure/ picotements : 5,2 % vs. 14,0 % à J15 (p=0,004), 6,8 % vs. 15,1 % à J42 (p=0,006), et 7,3 % vs. 19,9 % à J84 (p<0,001) et au prurit (1,5 % vs. 5,4 % à J42 et 2,4 % vs. 6,0 % à J84).

| Scores des symptômes subjectifs oculaires totaux liés à l'instillation à chaque visite (population pour l'évaluation de la tolérance et l'innocuité des collyres) | | | | |
|---|---|---|---|---|
| Visite | | EXEMPLE 1 | Xalatan | Valeur P* |
| J15 | n | 209 | 186 | 0,085 |
| | Moyenne ± DS | 0,25 ± 0,81 [0,14 ; 0,36] | 0,40 ± 0,89 [0,27 ; 0,53] | |
| | IC95 % | | | |
| J42 | n | 208 | 186 | 0,001 |
| | Moyenne ± DS | 0,15 ± 0,51 [0,08 ; 0,22] | 0,41 ± 1,03 [0,26 ; 0,56] | |
| | IC95 % | | | |
| J84 | n | 206 | 186 | 0,001 |
| | Moyenne ± DS | 0,18 ± 0,66 [0,09 ; 0,27] | 0,46 ± 1,05 [0,31 ; 0,61] | |
| | IC95 % | | | |

| | | | | |
|---|---|---|---|---|
| * ANOVA ajusté pour le pays | | | | |

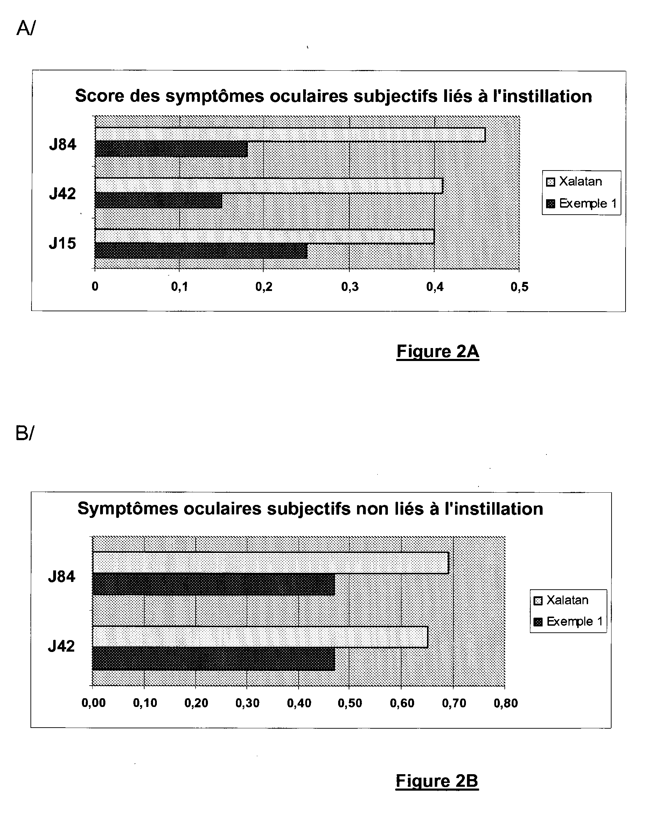

Ces résultats sont illustrés à la figure 2A.

### Symptômes subjectifs non liés à l'instillation

Globalement, il y a eu moins de symptômes oculaires subjectifs non liés à l'instillation dans l'oeil le pire du groupe EXEMPLE 1 comparé au groupe Xalatan à J42 (0,47 ±1,19 vs. 0,65 ± 1,54, p=0,057), et à J84 (0,47 ± 1,37 vs. 0,69 ± 1,73, p=0,053). Des résultats similaires ont été montrés pour les yeux adelphes.

Aussi bien dans l'oeil le pire que dans l'oeil adelphe, irritation/brûlure/picotements non liés à l'instillation ont été moins fréquents dans le groupe EXEMPLE 1 que dans le groupe Xalatan à J42 (7,2 % vs. 13,4 %) et à J84 (6,8 % vs. 12,9 %) dans l'oeil le moins beau. Ces résultats sont illustrés à la figure 2B.

Bien que ces différences se soient avérées statistiquement non significatives, il y a eu une tendance en faveur de l'EXEMPLE 1 qui confirme ce qui a déjà été observé pour les symptômes liés à l'instillation.

### Examen à la lampe à fente

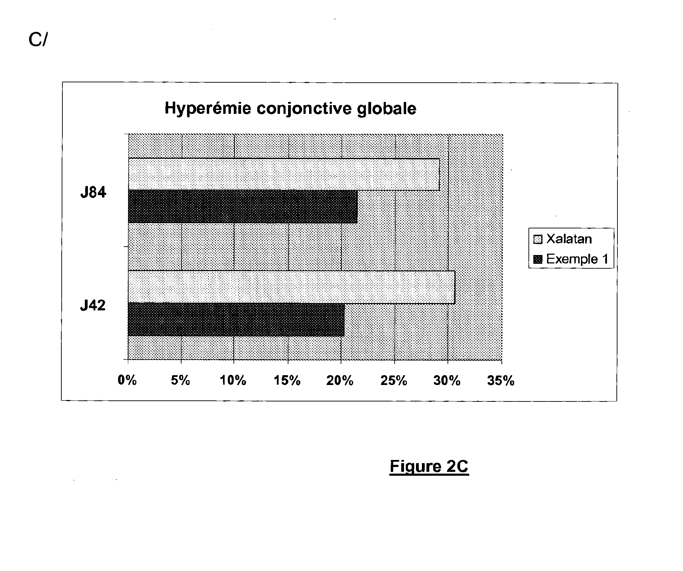

Globalement, l'hyperémie conjonctive a été moins fréquente dans le groupe EXEMPLE 1 que dans le groupe Xalatan dans l'oeil le pire avec une fréquence significativement plus basse dans le groupe EXEMPLE 1 que dans le groupe Xalatan à J42 (20,2 % vs. 30,6 %, p=0,003) et à J84 (21,4 % vs. 29,1 %, p=0,019). Ces résultats sont illustrés à la figure 2C. Pour chaque examen, aucune différence entre les traitements n'a été montrée.

### Appréciation globale par l'investigateur et par le patient :

Selon l'investigateur, la tolérance a été très satisfaisante ou satisfaisante pour plus de 97 % des patients dans les deux groupes de traitement. Cependant, le taux de tolérance très satisfaisante a été plus élevé dans le groupe EXEMPLE 1 que dans le groupe Xalatan à J15 (65,1 % vs. 59,7 %), à J42 (74,0 % vs. 65,1 %) et à J84 (71,4 % vs. 62,9 %). En considérant toutes les variables, des différences significatives ont été trouvées entre les 2 groupes de traitement à J42 (p=0,013) et J84 (p=0,047). Les résultats à J15 ne sont pas statistiquement significatifs.

A J42 et J84, plus de 99 % des patients du groupe EXEMPLE 1 et plus de 96 % des patients du groupe Xalatan ont trouvé le traitement adapté.

En conclusion, les résultats d'innocuité et de tolérance mettent en évidence une meilleure tolérance locale de l'EXEMPLE 1 par rapport à Xalatan, avec moins de symptômes subjectifs suite à l'instillation (en particulier prurit, et brûlure/picotements), et moins d'hyperémie. La tolérance a été notée plus fréquemment "très satisfaisante" chez les patients traités avec l'EXEMPLE 1.

En conclusion, cette étude démontre que la tolérance locale d'une composition selon l'invention (EXEMPLE 1) apparaît comme étant meilleure que pour Xalatan, avec au moins la même efficacité en termes de pression intra-oculaire.

## Revendications

1. Solution ophtalmique comprenant :
- au moins une prostaglandine ;
- un agent solubilisant ;
- un agent gélifiant de type carbomère ;
- un agent inhibant la polymérisation du carbomère ;
- un agent co-gélifiant / co-solubilisant ;
- ladite solution présentant une viscosité Brookfield à 25°C comprise entre 8 et 20 mPa.s ; et
- ladite solution étant dépourvue d'un agent conservateur de type anti-microbien.

2. Solution selon la revendication 1, **caractérisée en ce que** la viscosité est comprise entre 10 et 14 mPa.s.

3. Solution selon la revendication 1 ou 2, **caractérisée en ce que** l'agent de type anti-microbien est de type ammonium quaternaire, encore plus avantageusement de type chlorure de benzalkonium (BAK).

4. Solution selon l'une des revendications 1 à 2, **caractérisée en ce que** la concentration en agent gélifiant de type carbomère est comprise entre 0,05 et 0,15% (w/v).

5. Solution selon l'une des revendications 1 à 4, **caractérisée en ce que** la prostaglandine est choisie dans le groupe comprenant 17-phenyl-13,14 dihydro trinor Prostaglandin F_{2α} isopropyl ester (latanoprost), 20-ethyl Prostaglandin F_{2α}, (+)- Fluprostenol isopropyl ester (travoprost), 17-phenyl trinor Prostaglandin F_{2α} amide, 17-phenyl-13,14 dihydro trinor Prostaglandin F_{2α} ethyl amide (bimatoprost), tafluprost Prostaglandin F_{2α} ethanolamide, bimatoprost (freeacid)-d₄, bimatoprost-d₄, latanoprost ethyl amide, 13,14 dihydro-15-keto-20-ethyl Prostaglandin F_{2α} (unoprostone), 13,14 dihydro-15-keto-20-ethyl Prostaglandin F_{2α} isopropyl ester (unoprostone isopropyl ester), avantageusement le latanoprost.

6. Solution selon l'une des revendications 1 à 5, **caractérisée en ce que** la concentration en prostaglandines dans la solution est comprise entre 0,002 et 0,15 % (w/v).

7. Solution selon l'une des revendications 1 à 6, **caractérisée en ce que** l'agent inhibant la polymérisation du carbomère est une source d'ions sodium, avantageusement de l'EDTA de sodium, de l'acétate de sodium ou du chlorure de sodium.

8. Solution selon l'une des revendications 1 à 7, **caractérisée en ce que** l'agent solubilisant est l'hydroxystéarate de macrogolglycérol.

9. Solution selon l'une des revendications 1 à 8, **caractérisée en ce que** l'agent co-gélifiant / co-solubilisant est un polymère choisi dans le groupe : polyéthylèneglycol (PEG), alcool polyvinylique (PVA) ou polyvinylpyrrolidone (PVP).

10. Solution selon l'une des revendications 1 à 9, **caractérisée en ce que** la solution est stable au moins 18 mois à température ambiante (25 ou 30°C).

11. Solution selon l'une des revendications 1 à 10, **caractérisée en ce que** la solution est compatible avec les flacons à usage unique ou multidose réalisé en PEBD sans additifs.

12. Solution selon l'une des revendications 1 à 11, **caractérisée en ce qu'**elle contient en outre un agent anti-glaucomateux choisi dans le groupe comprenant les béta-bloquants, les inhibiteurs d'anhydrase carbonique et les agonistes alpha-adrénergiques.

13. Solution selon l'une des revendications 1 à 12, **caractérisée en ce qu'**elle contient en outre un additif choisi dans le groupe comprenant les agents isotonisants, les agents antioxydants et les systèmes tampons.

14. Solution selon l'une des revendications 1 à 13 pour son utilisation pour traiter le glaucome et/ou réduire la pression intraoculaire.

15. Solution selon la revendication 14 consistant à administrer à l'homme ou à l'animal, par voie topique, une goutte par jour de ladite solution dans chaque oeil.

16. Flacon à usage unique ou multidose réalisé en PEBD sans additifs comprenant la solution ophtalmique objet de l'une des revendications 1 à 13.

## Patentansprüche

1. Ophtalmologische Lösung, umfassend:
- wenigstens ein Prostaglandin;
- ein Lösungsvermittler;
- ein Geliermittel vom Typ Carbomer;
- einen die Polymerisation des Carbomers hemmenden Wirkstoff;
- ein Co-Geliermittel / Co-Lösungsvermittler;
- wobei die genannte Lösung bei 25°C eine zwischen 8 und 20 mPa.s inbegriffene Brookfield-Viskosität aufweist; und
- die genannte Lösung frei von einem Konservierungsmittel vom antimikrobiellen Typ ist.

2. Lösung gemäß Anspruch 1, **dadurch gekennzeichnet, dass** die Viskosität zwischen 10 und 14 mPa.s. inbegriffen ist.

3. Lösung gemäß Anspruch 1 oder 2, **dadurch gekennzeichnet, dass** der Wirkstoff vom anti-mikrobiellen Typ vom Typ quaternäres Ammonium, noch vorteilhafter vom Typ Benzalkonium-Chlorid (BAK) ist.

4. Lösung gemäß Anspruch 1 bis 3, **dadurch gekennzeichnet, dass** die Konzentration an Geliermittel vom Typ Carbomer zwischen 0,05 und 0,15 % (Gew. / Vol.) inbegriffen ist.

5. Lösung gemäß Anspruch 1 bis 4, **dadurch gekennzeichnet, dass** das Prostaglandin aus der Gruppe ausgewählt ist, die 17-Phenyl-13,14 Dihydro-Trinor-Prostaglandin F_{2α} Isopropyl-Ester (Iatanoprost), 20-Ethyl-Prostaglandin F_{2α}, (+)-Fluprostenol-Isopropyl-Ester (Travoprost), 17-Phenyl-Trinor-Prostaglandin F_{2α} Amid, 17-Phenyl-13,14-Dihydro-Trinor-Prostaglandin F_{2α} Ethyl-Amid (Bimatoprost), Tafluprost-Prostaglandin F_{2α} Ethanolamid, Bimatoprost (Freeacid)-d4, Bi-matoprost-d4, Iatanoprost-Ethyl-Amid, 13,14-Dihydro-15-Keto-20-Ethyl-Prostaglandin F_{2α} (Unoproston), 13,14-Dihydro-15-Keto-20-Ethyl-Prostaglandin F_{2α}-Isopropyl-Ester (Unoproston Isopropyl-Ester), vorteilhaft Iatanoprost, umfasst.

6. Lösung gemäß Anspruch 1 bis 5, **dadurch gekennzeichnet, dass** die Konzentration an Prostaglandinen in der Lösung zwischen 0,002 und 0,15 % (Gew. / Vol.) inbegriffen ist.

7. Lösung gemäß Anspruch 1 bis 6, **dadurch gekennzeichnet, dass** der die Polymerisation des Carbomers hemmende Wirkstoff eine Natriumionenquelle, vorteilhaft Natrium-EDTA, Natrium-Acetat oder Natrium-Chlorid ist.

8. Lösung gemäß Anspruch 1 bis 7, **dadurch gekennzeichnet, dass** der Lösungsvermittler Macrogolglycerol-Hydroxystearat ist.

9. Lösung gemäß Anspruch 1 bis 8, **dadurch gekennzeichnet, dass** das Co-Geliermittel / der Co-Lösungsvermittler ein Polymer ist, das aus der Gruppe: Polyethylenglycol (PEG), Polyvinylalkohol (PVA) oder Polyvinylpyrrolidon (PVP) ausgewählt ist.

10. Lösung gemäß Anspruch 1 bis 9, **dadurch gekennzeichnet, dass** die Lösung wenigstens 18 Monate bei Raumtemperatur (25 oder 30°C) stabil ist.

11. Lösung gemäß Anspruch 1 bis 10, **dadurch gekennzeichnet, dass** die Lösung mit den Einweg- oder Multidosen-Fläschchen kompatibel ist, das sich aus PEBD ohne Zusatzstoffe zusammensetzen.

12. Lösung gemäß Anspruch 1 bis 11, **dadurch gekennzeichnet, dass** sie darüber hinaus einen anti-glaukomatösen Wirkstoff enthält, der aus der Gruppe ausgewählt ist, die Betablocker, karbonische Anhydrase-Inhibitoren und alpha-adrenerge Agonisten umfasst.

13. Lösung gemäß Anspruch 1 bis 12, **dadurch gekennzeichnet, dass** sie darüber hinaus einen Zusatzstoff umfasst, der aus der Gruppe ausgewählt ist, die isotonisierende Wirkstoffe, antioxidierende Wirkstoffe und Puffersysteme umfasst.

14. Lösung gemäß Anspruch 1 bis 13 für ihre Verwendung zur Behandlung des Glaukoms und / oder zur Reduzierung des Augeninnendrucks.

15. Lösung gemäß Anspruch 14, bestehend aus der Verabreichung eines Tropfens der genannten Lösung in jedes Auge jeden Tag auf topischem Wege beim Menschen oder beim Tier.

16. Einweg- oder Multidosen-Fläschchen, der sich aus PEBD ohne Zusatzstoffe zusammensetzt und das die ophtalmologische Lösung gemäß Anspruch 1 bis 13 enthält.

## Claims

1. An ophthalmic solution including:
- at least one prostaglandin;
- a solubilizing agent;
- a gelling agent of the carbomer type;
- a carbomer polymerization-inhibiting agent;
- a co-gelling/co-solubilizing agent,
Wherein:
- the solution has a Brookfield viscosity at 25°C that is between 8 and 20 mPa.s;
- the solution contains no antimicrobial preservatives.

2. Solution as claimed in claim 1, **characterised in that** it has a Brookfield viscosity at 25°C that is between 10 and 14 mPa.s.

3. Solution as claimed in claim 1 or 2, **characterised in that** the antimicrobial preservatives is of the quaternary ammonium type, and even more advantageously benzalkonium chloride (BAK).

4. Solution as claimed in claim 1 or 2, **characterised in that** the concentration of the carbomer gelling agent is between 0.05 and 0.15% (w/v).

5. Solution as claimed in any of claims 1 to 4, **characterised in that** the prostaglandin is chosen from the group including 17-phenyl-13,14 dihydro trinor prostaglandin F_{2α} isopropyl ester (latanoprost), 20-ethyl prostaglandin F_{2α}, (+)- fluprostenol isopropyl ester (travoprost), 17-phenyl trinor prostaglandin F_{2α} amide, 17-phenyl-13,14 dihydro trinor prostaglandin F_{2α} ethyl amide (bimatoprost), tafluprost prostaglandin F_{2α} ethanolamide, bimatoprost (free acid)-d₄, bimatoprost-d₄, latanoprost ethyl amide, 13,14 dihydro-15-keto-20-ethyl prostaglandin F_{2α} (unoprostone), 13,14 dihydro-15-keto-20-ethyl prostaglandin F_{2α} isopropyl ester (unoprostone isopropyl ester), advantageously latanoprost.

6. Solution as claimed in any of claims 1 to 5, **characterised in that** the concentration of prostaglandins in the solution is between 0.002 and 0.15% (w/v).

7. Solution as claimed in any of claims 1 to 6, **characterised in that** the agent inhibiting the polymerization of the carbomer is a source of sodium ions, advantageously sodium EDTA, sodium acetate or sodium chloride.

8. Solution as claimed in any of claims 1 to 7, **characterised in that** the solubilizing agent is macrogolglycerol hydroxystearate.

9. Solution as claimed in any of claims 1 to 8, **characterised in that** the co-gelling / co-solubilizing agent is a polymer chosen from the group including polyethylene glycol (PEG), polyvinyl alcohol (PVA) or polyvinylpyrrolidone (PVP).

10. Solution as claimed in any of claims 1 to 9, **characterised in that** the solution is stable for at least 18 months at ambient temperature (25 or 30°C).

11. Solution as claimed in any of claims 1 to 10, **characterised in that** the solution is compatible with single-use or multi-dose bottles made of LDPE containing no additives.

12. Solution as claimed in any of claims 1 to 11, **characterised in that** it also contains an antiglaucoma agent chosen from the group including beta blockers, carbonic anhydrase inhibitors and alpha-adrenergic agonists.

13. Solution as claimed in any of claims 1 to 12, **characterised in that** it also contains an additive chosen from the group including isotonic agents, antioxidants and buffer systems.

14. Solution as claimed in any of claims 1 to 13 for its use in treating glaucoma and/or reducing intraocular pressure.

15. Solution as claimed in claim 14 consisting in topically administering one drop of said solution a day to each eye in humans or animals.

16. Single-use or multi-dose bottle made of LDPE containing no additives containing the ophthalmic solution covered by any of claims 1 to 13.
